(19) **Europäisches Patentamt**
**European Patent Office**
**Office européen des brevets**

(11) **EP 2 613 141 A1**

(12) **EUROPEAN PATENT APPLICATION**
published in accordance with Art. 153(4) EPC

(43) Date of publication:
**10.07.2013 Bulletin 2013/28**

(51) Int Cl.:
*G01N 27/62* *(2006.01)*

(21) Application number: **11821333.9**

(22) Date of filing: **31.08.2011**

(86) International application number:
**PCT/JP2011/004884**

(87) International publication number:
**WO 2012/029312 (08.03.2012 Gazette 2012/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.08.2010 JP 2010193382**
**31.08.2010 JP 2010193361**

(71) Applicant: **Atonarp Inc.**
**Tokyo 192-0074 (JP)**

(72) Inventors:
• **IMAI, Akira**
**Tsukuba-shi**
**Ibaraki 305-0047 (JP)**

• **SATO, Tomoyoshi**
**Tsukuba-shi**
**Ibaraki 305-0047 (JP)**
• **MURTHY, Prakash Sreedhar**
**Tsukuba-shi**
**Ibaraki 305-0047 (JP)**

(74) Representative: **Körber, Martin Hans**
**Mitscherlich & Partner**
**Sonnenstraße 33**
**80331 München (DE)**

(54) **METHOD AND APPARATUS FOR FINDING CANDIDATE FROM DATA MEASURED BY SENSOR**

(57)    There is provided an apparatus (10) including:
an interface (13) that receives a first measured data set
(51) measured by a FAIMS (1) and first environment information (60) indicating the measurement environment
of the first measured data set; a generation unit (200)
that generates a plurality of virtual data sets (71) by converting a plurality of existing data sets (81) from a database (80) using conditions controlled according to the
first environment information (60); and an output unit (30)
that selects and outputs at least one candidate that is
close to the first measured data set (51) from a provisional
group including the plurality of virtual data sets (71).

Fig. 4

**Description**

TECHNICAL FIELD

The present invention relates to find out of candidates that have been measured by a sensor based on data obtained by a sensor such as a DIMS.

BACKGROUND ART

Field Asymmetric waveform Ion Mobility Spectrometers (FAIMS) and Differential Ion Mobility Spectrometers (DMS) are known as sensors for detecting substances in air. A spectrometer (or sensor, hereinafter collectively referred to as "DMS") of this type inputs an ionized fluid sample (as examples, gas, liquid, or vapor) into an asymmetrical electrical field that changes from high voltage to low voltage and outputs the result of filtering such flows based on the field mobility of ions.

Japanese Patent Publication No. 2008-508693 (W02006/013396 , hereinafter referred to as "Document 1") discloses an apparatus for measuring physical phenomena caused by differences in ion mobility between substances. In particular, Document 1 describes an ion mobility spectrometer having an ion filter in the form of at least one ion channel with a plurality of electrodes. With this ion mobility spectrometer, it is possible for the filler to selectively admit ion species according to a time-varying potential applied to the conductive layers. Such potential has a drive field component and a transverse field component, and in a preferred embodiment, the respective electrodes contribute to the generation of components of both the drive field and the transverse field. Such device may be used even without a drift gas flow. In addition, Document 1 discloses a micromachining technology for fabricating a microscale spectrometer for the various applications of a spectrometer.

Japanese Patent Application No. 2007-513340 (WO2005/052546, hereinafter "Document 2") discloses a technology relating to an ion mobility-based system, method, and apparatus for analyzing samples in general. As one example, a number of improvements are disclosed for sample collection, filtering, detection, measurement, identification, and/or analysis using dispersion characteristics, sample fragmentation, and/or variations in sample processing, such as variations in the flow channel/filtering electric field state. Such conditions are described as including pressure, temperature, humidity, the strength and duty cycle and/or frequency of the electric field, the electric field voltage amplitude and frequency and/or duty cycle, the bias voltage magnitude and/or polarity of the detector, and/or the magnitude and/or polarity of the filtering electric field compensation voltage.

For DMS, it is known that two-dimensional spectra are obtained by changing the compensation voltage Vc (Vcomp) relative to the field voltage Vrf and that three-dimensional spectra are obtained by further changing the field voltage Vrf. Information on an ionized fluid sample (hereinafter referred to in general as a "gas") to be measured by DMS is included in such two-dimensional or three-dimensional spectra, and it should be possible to specify gas constituents by analyzing the spectra. In Document 2, a library of spectral signatures for a plurality of known species is provided and an attempt is made at identifying an unknown species by comparing at least part of a spectral signature of such unknown species with one or more spectral signatures stored in the library.

Document 2 also states that a spectral signature includes a spectral peak amplitude, spectral peak width and spectral peak slope, spectral peak spacing, spectral peak quantity, relative shifts of spectral peaks due to changes in processing conditions, spectral discontinuities, a Vrf versus Vcomp characteristic, or the result of plotting an arbitrary characteristic aside from the above conditions relative to at least one other arbitrary condition.

Such information includes information on ion mobility that is unique to the gas to be measured or its constituents. However, it is also known that the appearance of information on ion mobility changes according to pressure, temperature, humidity, and the like.

It is also known that the appearance of information will change depending on the apparatus, and Document 2 states that reliable identification of a detected species is achieved using each of multiple data via the inherent mobility characteristic that identifies such species. According to one embodiment, it is possible to compare against a lookup library specific to the apparatus in question. It is also possible to compare against a common data set that is not dependent on the apparatus. In addition, Document 2 discloses that a system is calibrated before being used to analyze samples. More specifically, a library of ion intensities for known ion species at particular Vcomp and Vrf settings is created and stored in a memory. According to one embodiment, once the system has been calibrated, it is possible to continuously use the system without needing further calibration. Document 2 states that as examples, a reactant ion peak (RIP) or a dopant peak is used to calibrate the system.

In addition, Document 2 states that in order to determine the degree of match between a data object (reference vector) P and the measurement value vector P', as one example, if the elements (Vcomp, a) of P and P' are considered to be data points in a Euclidean geometry space, a distance can be computed and the comparison with the smallest Euclidean distance is then selected as the best match. The document also states that it is possible to find a best match for P' using a known pattern recognition algorithm, a neural network, or artificial intelligence technology.

DISCLOSURE OF THE INVENTION

Even though such methods have been disclosed for a DMS, it is not possible to claim that technologies for specifying a gas or the constituents of a gas using a DMS are in widespread use.

One aspect of the present invention is an apparatus including: an interface that receives a first measured data set that is measured by a first sensor and first environment information showing a measurement environment of the first measured data set; a generation unit that generates a plurality of virtual data sets by converting a plurality of existing data sets from a database using conditions controlled according to the first environment information; and an output unit that selects and outputs at least one candidate that is close to the first measured data set from a provisional group including the plurality of virtual data sets.

Another aspect of the present invention is a method of having a processing apparatus process data measured by a first sensor, including steps of:

receiving a first measured data set that is measured by the first sensor;
generating a plurality of virtual data sets by converting a plurality of existing data sets from a database using conditions controlled according to the first environment information; and
selecting at least one candidate that is close to the first measured data set from a provisional group including the plurality of virtual data sets.

The appearance pattern of characteristics (features) of a measured object (gas or constituents of a gas) in a data set obtained by the first sensor such as a DMS is influenced by various elements (factors, causes). Accordingly, there is a low probability of a data set that has been influenced by such elements being included in the existing data sets. In addition, there is low probability that a data set showing the measured object included in the existing data sets will be a data set measured with extremely close conditions to a measured data set that has been influenced by a variety of elements. Accordingly, with this method, the number of data sets that are close to the measured data set is forcibly increased by generating virtual data sets, and as a result the size of the search space is reduced and the probability that a candidate found or selected using the measured data will be a favorable candidate is increased.

One method of generating virtual data sets is to convert a plurality of existing data sets using a model that has the first environment information as parameters. One example of a model is a function model that has a plurality of elements included in the environment information as parameters.

Another method of generating the virtual data sets is to stochastically combine a plurality of existing data sets. It is possible to stochastically generate virtual data sets even when the respective elements make it difficult to imagine or estimate the appearance pattern of a data set showing the measured objects included in the existing data sets, when all the elements that affect the appearance pattern cannot be obtained in advance, or when all of the elements that affect the appearance pattern and the effects caused by such elements are not all clearly understood.

In the method that is an aspect of the present invention, search objects (a search space) are virtually widened by stochastically combining a plurality of existing data sets, virtual data sets that are close to the measured data set are stochastically increased, and the measured object or a virtual data set that is close to the measured object is disposed closed to the measured data set. By then searching such a search space, there is the possibility of obtaining the measured object or a candidate that is close thereto and possible to improve the estimation probability for measured objects even if all of the elements that affect the appearance pattern cannot be obtained in advance and all of the elements that affect the appearance pattern and the effects due to such elements are not all clearly understood.

In addition, this method should preferably include a step of receiving first environment information showing the measurement environment of the first measured data set, and the process of generating the plurality of virtual data sets should preferably include generating the plurality of virtual data sets by combining a plurality of existing data sets with probabilities controlled according to the first environment information. Since there is the possibility of the range for generating the virtual data sets being limited by the first environment information, it is possible to narrow the search space.

BRIEF DESCRIPTION OF THE DRAWINGS

FIG. 1 is a block diagram showing an overview of a system.
FIGS. 2(a) and (b) show how measurement data changes.
FIG. 3 is a block diagram showing another example of a system.
FIG. 4 is a flowchart showing an overview of an estimation method.

DETAIL DESCRIPTION

The present invention will now be described in more detail for an example of a system that estimates gas constituents (components, "measured objects" or simply "objects) from data obtained by a FAIMS that is one example of a DMS that measures ion mobility of gas constituents included in air or a gas.

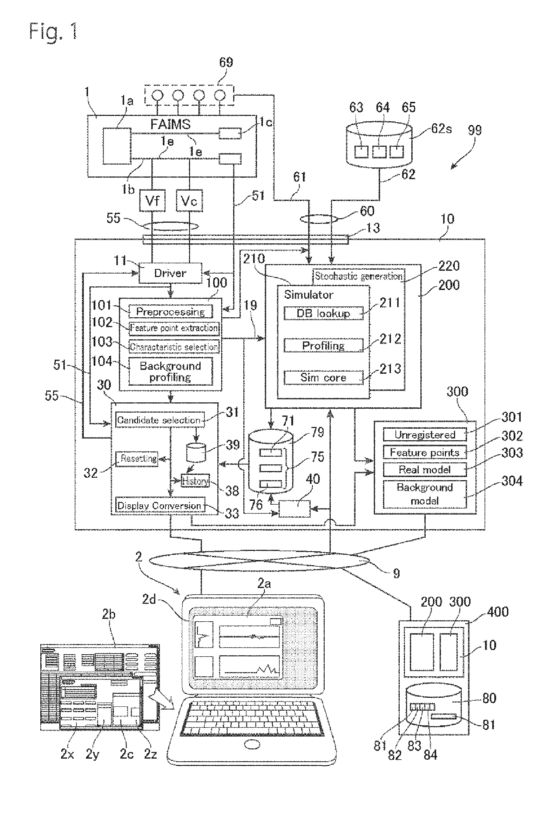

FIG. 1 shows the overall architecture of a processing apparatus (system) that analyzes data obtained from a FAIMS. Such processing apparatus 10 may be realized by a system connecting an appropriate communication means, for example a server connected by the Internet 9, to a sensor (FAIMS) 1 or a terminal equipped with the sensor 1, may be incorporated in the terminal, may be realized by software (a program or a program product), or may be partly or entirely realized by hardware (a semiconductor processing apparatus or processing apparatus) such as an LSI or an ASIC.

The FAIMS 1 includes an ionizing unit 1a that ionizes a target chemical substance (or "measured object" or "object"), a drift chamber 1b that transfers the ionized measured object while influencing the ionized measured object with an electric field, and a detector 1c that detects the ionized measured objects (electric charge of the objects, ion current) that has passed through the drift chamber 1b. In the drift chamber 1b, the electric field that is generated by the electrodes 1e and is controlled by software varies between positive and negative with a specified cycle, and due to the filtering effect of such electric field, chemical substances that are the detection targets are filtered, collide with the detector 1c, and are measured as electric currents in a short period, for example, at msec (millisecond) level.

Conversely, chemical substances aside from the measured objects can be subjected to a filtering process that changes the parameters of the electric field in the drift chamber 1b using software so that such chemical substances are not sent to the detector 1c. That is, with the FAIMS 1, it is possible to carry out detection and analysis (checking) collectively in short time. By executing a FAIMS 1 scan within a given measurement range, it is possible to analyze a wide range of chemical substances. In particular, due to the recent progress in MEMS technology, miniaturization has advanced and it has become possible to mount the FAIMS 1 in the preprocessing part of a recent LC (liquid chromatography) or MS (mass spectrometry) analyzer. By combining software technology and parallel processing technology in the future, the miniaturization of analyzers is expected to proceed further, which will result in widespread application in a variety of industrial fields, the medical field, and the home.

One example of a FAIMS 1 is a sensor made by Owlstone, with Ni63 (a 555MBq βsource, 0.1μSv/hr) being used in the ionizing unit 1a. Chemical substances that can be ionized by the ionizing unit 1a have an ionization binding energy of 67KeV or below, which means that it is possible to detect and analyze a wide range of chemical substances. Devices that use UV and devices that use corona discharge are being investigated as the ionizing unit 1a.

The FAIMS 1 is optimal as a platform for developing products that detect explosives and hazardous materials, detect threats, detect and analyze chemical substances, and detect, in real time, environmental chemical substances whose target changes. In particular, a FAIMS has a superior performance for detection of trace substances at a ppm/ppb/ppt level. If the target substances are distinct and the number of targets is 100 or below, in principle it should be possible to specify substances in 1 to 2 seconds. However, since the ease of measurement will change according to environmental conditions, it is necessary to confirm the background conditions. Accordingly, one important issue when using the FAIMS 1 in an actual application is to stably carry out detection and analysis of the target ("objects" or "chemical substances") with respect to background noise and the like that changes according to the state and differences in various environmental variables, so as to improve precision and also operate in real time or at a speed that is close to real time.

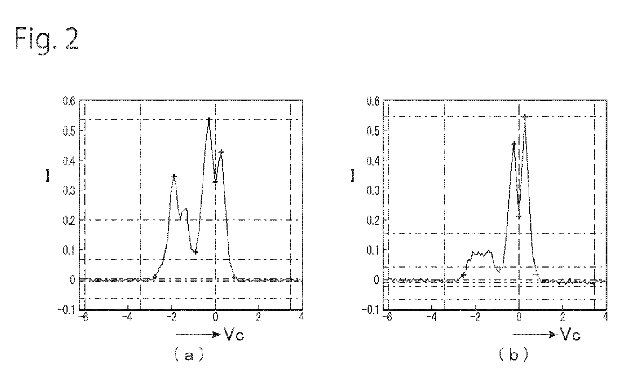

Examples of the measurement results of the FAIMS 1 are shown in FIGS. 2A and 2B. Even with the same sample, the measurement results of the FAIMS 1 can appear to exhibit different behavior according to the measurement environment. The processing apparatus 10 shown in FIG. 1 attempts to solve this problem by carrying out data processing in real time or at a speed close to real time using SW (software) processing technology. The functions of the processing apparatus 10 can be provided for example by one or a plurality of semiconductor integrated circuits. Such processing apparatus 10 is referred to as an OLP (OLfaction Processor) and is being developed by the present applicant as a platform for a technology to realize the real time detection, analysis, and database registration of chemical substances. Such OLP is expected to provide a platform that will enable many companies to carry out product development for expanding future markets in a short time, with high efficiency, and with high quality. Various systems 99 and services that include the FAIMS 1 can also be developed.

The OLP 10 includes an interface 13 that sets measurement conditions 55 in the FAIMS (sensor) 1 and acquires a measured data set (a first measured data set, hereinafter "IMS data") 51 from the sensor 1, and a driver 11. The measurement conditions 55 are sent from the driver 11 of the OLP 10 via the interface 13 to the sensor 1. The interface 13 may be wired or may be wireless, and may be digital or may be analog. An environment where the sensor 1 autonomously sets the measurement conditions 55 and the OLP 10 automatically acquires the IMS data 51 via the interface 13 is also possible.

The field voltage Vf (hereinafter simply "voltage Vf) and the compensation voltage Vc are included in the measurement conditions 55. One example of the IMS data 51 is a spectrum expressed by a current I (the current (iron current) detected by the detector 1c) that changes corresponding to variations in the compensation voltage Vc at a specified voltage Vf. The IMS data 51 may be data produced by sampling (extracting) feature points (characteristic points) of the spectrum described above or may include spectra for a plurality of voltages Vf. Since there is the possibility that the amount of data included in a spectrum will fall due to the extraction of feature points (although this depends on the communication speed), the IMS data 51 acquired by the OLP 10 should preferably be data produced by AD conversion of the spectrum acquired by the sensor 1.

The OLP 10 further includes a unit 100 that preliminarily analyzes the acquired IMS data 51, a generation unit (generator) 200 that acquires a plurality of existing data sets 81 from a database 80 via a network 9 or the like and generates a plurality of virtual data sets 71, a selection unit (selector) 40 that logically selects appropriate real data sets 76 from the existing data sets 81, an output unit 30 that selects one or a plurality of candidate data sets 75 that are close to the IMS data 51 out of the candidate data sets 75 in a candidate database 79 including the virtual data sets 71 and the real data sets 76, and a registration unit 300 that registers the measured IMS data 51 in a database 80 as an existing data set 81.

The respective existing data sets (already known data sets, already fixed data sets) 81 include results (data) produced by measuring constituents (elements, components) and/or products (commercial products) in the past using various types of IMS (ion mobility sensors, ion mobility spectrometers), results of calculating ion mobility from an existing chemical database (chemical substance database), and the like, and show existing data that is capable of being compared with measurement results ("measured data sets", "IMS data") 51 of the sensor 1 (a FAIMS in the present embodiment) being used for measurement. An existing data set 81 includes spectrum information 82, identification information 83 showing characteristics of a specified chemical substance, and variation information 84. The spectrum information 82 is information for generating a spectrum for comparing with the IMS data 51 obtained from the sensor 1 (a spectrum expressed by variations in the compensation voltage Vc at a specified voltage Vf) and includes ion mobility, spectrum samples for a representative voltage Vf of a typical sensor type, and the like. The identification information 83 includes the main name, chemical information (a CAS registration number, PUBCHEM, an RTECS number, chemical formula, molar mass, density, other physical properties, or the like), the possibility of being included in a product and proportion for such case, and if the chemical is a product (good), the constituents included in the product, proportions thereof, and the like. The variation information 84 includes information showing theoretical and/or experimental dependence on environment information (temperature, humidity, pressure, flow rate, electric field strength, sensor type, and the like) when substances appear as the IMS data 51.

The OLP 10 receives environment information (first environment information) 60 showing (including, identifying) the measurement environment obtained by the IMS data 51 from sensors or the like that has been set. The environment information 60 includes physical information 61 such as temperature, humidity, pressure, and flow rate, and application information 62. The application information 62 can be acquired from an application service server 62s that provides applications, the terminal in which the FAIMS 1 is incorporated, and the like. The application information 62 includes position information 63, image information 64, and user input information 65. It should be noted that the environment information 60 acquired by the OLP 10 may include some or all of such information.

The physical information 61 can be acquired from a sensor group 69 that is set or provided on the terminal in which the FAIMS 1 is installed or provided in the vicinity of the FAIMS 1. The sensor group (second sensors) 69 include sensors suited to measuring physical quantities (physical values) such as temperature, humidity, pressure, and flow rate. The environment information 60 for the periphery of the FAIMS 1 can also be indirectly acquired from another system that makes fixed-point observations of temperature, humidity, and the like using the application information 62, such as the position information 63. In addition, if the time and date are included in the environment information 60, it is possible to approximately estimate the temperature range from the time and date and use such value as the physical information 61.

The generation unit 200 includes a simulator 210 that generates virtual data sets 71 using a model, and a stochastic generation unit 220 that stochastically generates the virtual data sets 71. Hereinafter, the description will focus on the case where the virtual data sets 71 are mainly generated by the simulator 210.

The IMS data 51 acquired via the driver 11 is first subj ected to preliminary analysis by the preliminary analysis unit 100. The preliminary analysis unit 100 includes a preprocessing unit 101 that carries out processing to remove background noise included in the spectra in the IMS data 51, to separate a plurality of peaks, to acquire parameters that characterize the respective peaks, and the like. The preliminary analysis unit 100 also includes a feature point extracting unit 102 that extracts a number of points (feature points) that characterize the IMS data 51 out of the data obtained by the preprocessing unit 101, a feature selecting unit 103 that selects, from the extracted feature points, a profile thought to be the characteristics of chemical substances that are (candidate) sources for the IMS data 51, and a background profiling unit 104 that stores a background profile of the IMS data 51 as supplementary information.

The feature point extracting unit 102 extracts the positions (the relationship with the field voltage Vf and the compensation voltage Vc), the intensities, the order, and the like of the separated peaks as feature points. From a number of feature points, the feature selecting unit 103 theoretically, experimentally, and empirically (i.e., through learning) selects a profile thought to be the characteristics of chemical substances that are (candidate) sources for the IMS data 51. The background profiling unit 104 stores a background profile produced by subtracting the profile selected as the characteristics of the IMS data 51 from the IMS data 51 as supplementary information for increasing and/or changing the candidate variations.

The simulator 210 includes a reference unit 211 that refers to the existing database 80 based on feature point information 19 acquired by the preliminary analysis unit 100 and selects a plurality of data sets that share feature points and the like out of the existing data set 81, a profiling unit 212 that generates parameters to be used in a prediction model from the feature point information 19 obtained by the preliminary analysis unit 100 and the environment information 60, and a simulator core 213 that generates virtual data sets 71 using the generated parameters and the prediction model.

The OLP 10 includes a unit 40 that uses the feature point information 19 to select real data sets 76 with common feature points from the existing database 80. When a measured data set is compared with only real data sets 76, it is not possible to increase the number of data by adding extra feature points and it is also not possible, when the difference between real data sets is small, to reduce the Euclidean distance between the IMS data 51 and the real data sets 76 when such distance is large. In addition, even in chemical substances that are very similar, if the concentration and/or temperature and electric field strength are changed, the difference in ion mobility will change as described earlier.

By actively changing the existing data sets 81 using environmental variables (environmental conditions) 60, the simulator 210 constructs a database 79 for specifying chemical substances that are (candidate) sources for the IMS data 51.

For the prediction model used by the simulator core 213, it is known that ion mobility depends on the cross-sectional area and molecular structure of respective chemical substances. Accordingly, if the functional group to which a chemical substance belongs can be determined from the feature point information 19, it is possible to improve the precision of the prediction model and to greatly reduce the search space. In addition, the prediction model should preferably have the cross-sectional area, molecular weight, ionization amount, concentration, temperature, humidity, background material, and the like of a chemical substance as parameters. Ion mobility often exhibits nonlinear behavior with respect to such parameters. Accordingly, although the prediction model may include linear interpolation or extrapolation and interpolation, it is preferable to use a nonlinear functional model with fast convergence. To enable use as a prediction model, a model should preferably be capable of easily absorbing deviations from the measurement data. One candidate for a functional model is a model that uses a spline function. The functional model may be a nonlinear series (function) that has environmental conditions as parameters. The simulator core 213 may also have a regression convergence function. The prediction model should preferably be able to cause convergence to data included in a plurality of existing databases 80 present on the Internet 9.

By doing so, in the OLP 10, a plurality of candidate data sets 75 that are groups of a plurality of virtual data sets 71 generated by the generation unit 200 and one or a plurality of real data sets 76 theoretically generated by the selection unit 40 are generated, thereby producing the candidate database 79. Here, there is the possibility that the plurality of virtual data sets 71 generated by the generation unit 200 will have a shorter Euclidean distance from the IMS data 51 than the real data sets 76. Accordingly, there is the possibility that it will be possible to specify the chemical substances that are the source of the IMS data 51 with much higher precision. Note that the stochastic generation unit 220 will be described in detail later in this specification.

The output unit 30 includes a candidate selection unit 31 that selects one or a plurality of candidate data sets 75 that are estimated with a certain probability of being a source (object) of the IMS data 51, a sensor control unit 32 that resets the measurement conditions of the sensor 1, a database or cache 39 that stores a history of candidates selected in the past, a history searching unit 38 that refers to the history and selects candidates expected to have the highest probability out of a plurality of candidates, and a display conversion unit 33 that converts the selected candidate data sets 75 to constituent names or product names and transmits to a terminal 2 of the user.

The candidate selection unit 31 determines the distances between the IMS data 51 and the respective candidate data sets 75 of the candidate database 79 for a larger number of points than the feature point information 19 and selects one or a plurality of candidate data sets 75 that are estimated with a certain probability of being an object of the IMS data 51. If there are measurement conditions for the sensor 1 that are capable of changing the rankings of a plurality of candidate data sets 75 that have the same probabilities (ranks), the sensor control unit 32 is capable of sending such conditions 55 back to the driver 11, having measurement repeated by the sensor 1, and acquiring IMS data 51 where an optimal candidate is selected out of a plurality of candidates.

The history searching unit 38 selects and outputs common candidate data sets 75 that have been selected for the IMS data 51 obtained immediately previously or consecutively out of the plurality of candidate data sets 75 included in the history recorded in the cache 39. As one example, the history searching unit 38 outputs candidate data sets 75 that are common to both a plurality of candidate data sets 75 selected from first IMS data 51 and first environment information 60 and a plurality of candidate data sets 75 selected from second IMS data 51 and second environment information 60 as the candidate data sets 75 with the highest probability.

The display conversion unit 33 selects or generates content including constituent names or product names from the identification information 83 of the existing data set 81 combined with the candidate data sets 75 and provides the candidate data sets 75 that are close to the IMS data 51 to the terminal 2 of the user as content. The display conversion unit 33 provides the candidate data sets 75 as information that can be displayed on a display apparatus 2d of the terminal 2. If a candidate data set 75 is a recipe or the like of a perfume and is protected by a copyright, a patent, or some other type of rights, the display conversion unit 33 has a product name, not constituent names, displayed on the display apparatus 2d.

The OLP 10 further includes the registration unit 300 that registers the IMS data 51 in the existing database 80. The registration unit 300 includes a unit 301 that determines whether a chemical substance that is a source of the IMS data 51 is unregistered. When the Euclidean distance between the IMS data 51 and the data included in the candidate database 79 is not within a specified range at the output unit 30, the unit 301 determines that an existing data set 81 that produces the IMS data 51 is not included in the database 80. The IMS data 51 that has been determined to be unregistered is registered in the existing database 80 together with a number of candidate data sets 71 and 76 whose Euclidean distances are near in readiness for resolution at a future date when the amount of data in the existing database 80 has increased and/or the prediction model of the simulator 210 is improved.

The registration unit 300 also includes a unit 302 that registers the IMS data 51 in the database 80 in association with the feature point data 19, a unit 303 that registers the IMS data 51 in the database 80 as a real model, and a unit 304 that registers the IMS data 51 as a background model. The unit 303 that registers the IMS data 51 as a real model registers the IMS data 51 in the database 80 as representative data (or a "real model") of a chemical substance, constituent, or product that has been specified from the IMS data 51. The unit 304 that registers the IMS data 51 as a background model registers the IMS data 51 in the database 80 as supplementary data of a chemical substance, constituent, or product that has been specified from the IMS data 51.

As examples, the FAIMS 1 and the OLP 10 shown in FIG. 1 are a mobile observation system, a fixed point observation system, or a remote observation system connected to the network 9. The display conversion unit 33 has a function which, via the network 9, has the terminal 2, such as a personal computer, display the IMS data 51 as an image 2a, display the data of the cache 39 as an image 2b, and also display the content 2c. The content 2c includes a plurality of information 2x that is openly available on the network 9 corresponding to the plurality of candidate data sets 75, information 2y on the most probable candidates, and information 2z relating to the information 2y on the most probable candidates.

The functions as the OLP 10 are capable of being provided in a server 400 that provides a service as the existing database 80, with it also being possible for the server 400 to provide the content 2c to the terminal 2 via the network 9.

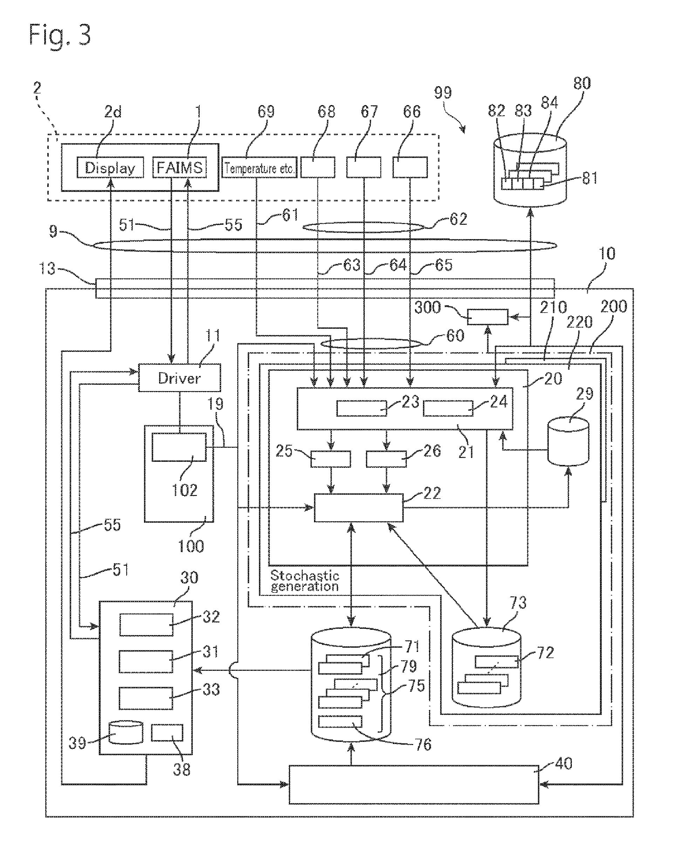

FIG. 3 schematically shows a system 99 where a sensor (FAIMS) 1 is installed in the terminal 2. The OLP 10 that is an apparatus (system) for analyzing the data of the FAIMS 1 may be realized by a server with a suitable communication means, for example a server connected by the Internet 9 to the terminal 2 equipped with the sensor 1 or may be incorporated in the terminal 2.

The terminal 2 may have an environment capable of remote sensing where the measurement conditions 55 of the sensor 1 are provided via the interface 13 from the driver 11 of the OLP 10. The terminal 2 may use an environment where the terminal 2 autonomously sets the measurement conditions 55 and the OLP 10 is capable of automatically acquiring the IMS data 51 and the measurement conditions 55 via the interface 13.

As described earlier, the OLP 10 includes the preliminary analysis unit (analyzer) 100, the generation unit (generator) 200, the selection unit (selector) 40, and the registration unit 300. The generation unit 200 includes the simulator 210 and the stochastic generation unit 220 that acquires, via the network 9 or the like, a plurality of existing data sets 81 from the database 80 and stochastically generates the plurality of virtual data sets 71. The following description will focus on an example where the virtual data sets 71 are stochastically generated.

The OLP 10 receives the environment information (first environment information) 60 showing the measurement environment at the time when the IMS data 51 is obtained, from the terminal 2, a sensor set in the periphery of the terminal 2, or the like. The physical quantity information 61 on the temperature, humidity, pressure, flow rate and the like in the environment information 60 can be acquired from the sensors ("second sensors" or "sensor group") 69 installed in the terminal 2. As described above, the physical quantity information 61 may be acquired indirectly from another system that makes fixed-point observations of temperature, humidity, and the like based on the position information 63 of the terminal 2.

The application information 62 is information used to narrow the search range for objects measured by the sensor 1. The position information 63 is obtained from GPS (or "third sensor") 68 installed in the terminal 2, information of base stations of mobile phones, or the like. Obtained position information 63 may become a base for estimating objects (chemical substances) with a high probability of being measured by the FAIMS 1 and thereby narrow the search range. The image information 64 is typically obtained from a camera 67 installed in the terminal 2. Alternatively, based on the position information 63, it is also possible to obtain an image showing the periphery of the position of the terminal 2 from a fixed point monitor. The image information 64 is used to estimate a range of objects with a high probability of being measured by the sensor 1.

The user input information 65 is capable of being acquired from a user interface (external input interface) 66 of the terminal 2 or from user information on the Internet (or "in the cloud"). The user input information 65 includes various grades of information, which include information on the field in which the user wants information, as examples, health, food, drinks, and animals, and by combining such information, it is possible to estimate the range of objects that have a high probability of being measured by the sensor 1.

The stochastic generation unit 220 of the generation unit 200 includes a stochastic processing unit 20, a library in which functions, models, initial values and the like referred to by the processing unit 20 are stored, and a provisional candidate database 73 that is a work area of the processing unit 20. The processing unit 20 includes a stochastic combining engine 21 and an iterative algorithm engine 22. The processing unit 20 of the stochastic generation unit 220 generates virtual data sets 71 by stochastically combining the existing data sets 81 obtained from the database 80. In this example, the processing unit 20 generates the virtual data sets 71 in two stages. First, the stochastic combining engine 21 stochastically combines a plurality of existing data sets 81 based on the environment information 60 to generate the provisional candidate database 73 including the plurality of provisional candidate data sets 72 used by the iterative algorithm engine 22. The stochastic combining engine 21 also generates one or a plurality of functions (initial functions) 25 for combining the provisional candidate data sets 72 and one or a plurality of initial conditions 26.

Next, the iterative algorithm engine 22 stochastically discovers or finds one or a plurality of virtual data sets 71 that are close to the IMS data 51 according to a meta-heuristic method using the feature point information 19 of the IMS data 51 acquired by the preliminary analysis unit 100 as a target. Meta-heuristic iterative algorithms may be simulated annealing, mean field annealing, genetic algorithms and others.

The stochastic combining engine 21 combines the existing data sets 81 with a probability that is controlled according to the environment information 60 to narrow the search range used in subsequent processing. More specifically, the stochastic combining engine 21 generates a plurality of provisional candidate data sets (first converted data sets) 72 by theoretically and/or experimentally changing the existing data sets 81 based on the environment information 60 and in particular on the physical information 61 such as temperature. To theoretically and/or experimentally change the existing data sets 81 based on the physical information 61, the stochastic combining engine 21 can use the variation information 84 of the existing data set 81.

In theory, the average movement velocity vd of the ions of each molecule is proportional to the strength of the electric field E and is given by the following equation (1).

$$vd = K \times E \ \dots \ (1)$$

One example of the theoretical formula of a motion coefficient K that provides ion mobility is given as follows according to the momentum transfer theory.

$$K = (3e/16N) \times (2\pi/\mu \cdot k \cdot Teff)^{1/2} ((1+\alpha)/\Omega d(Teff)) \ \dots \ (2)$$

Here, e is the charge of an ion, N is the density of the buffer gas, k is Boltzmann's constant, $\mu$ is the reduced mass of the ion and the buffer gas molecules, Teff is the effective temperature of the buffer gas in the drift tube, and $\alpha$ is a correction term that is normally much lower than 1. $\Omega$d is the collision cross section (collision integral) and is given by the following formula (3).

$$\Omega d = \pi r^2 \Omega(1,1) \times T \ \dots \ (3)$$

Here, r is the radius of a molecule, and Q(1,1) is a value that is specific to the molecule and changes according to humidity, temperature, buffer gas (carrier gas, drift gas) and the like.

Accordingly, the stochastic combining engine 21 is capable of using such theoretical formulas and the results of analyzing a large number of experimental results based on the theoretical formulas described above to change the existing data sets 81 according to the environment information 60.

The stochastic combining engine 21 is also capable of generating a plurality of provisional candidate data sets (or "second converted data sets") 72 by combining a plurality of data sets out of the existing data sets 81 based on the environment information 60 (and in particular the application information 62) so that the probability becomes relatively higher. To combine the existing data sets 81 according to the application information 62, the identification information 83 of the existing data sets 81 can be used. In addition, by incorporating the feature point information 19 of the IMS data 51 in the initial values or the like, the stochastic combining engine 21 attempts to set the range for stochastic estimation close to the IMS data 51.

In the processing unit 20, the stochastic combining engine 21 generates the provisional candidate data sets 72 by stochastically combining the existing data sets 81 based mainly on the application information 62. Next, the initial functions 25 and the initial conditions 26 used in the iterative algorithm engine 22 are generated based on the physical information 61. In addition, the iterative algorithm engine 22 combines the provisional candidate data sets 72 via theoretical and/or experimental parameters in an attempt to stochastically discover a plurality of provisional candidate data sets 71 that are close to the IMS data 51.

The stochastic combining engine 21 includes one or a plurality of combining engines. In the present example, the stochastic combining engine 21 includes a Bayesian combining engine 23 and a heuristic combining engine 24, with such engines being used separately or in combination. The heuristic combining engine 24 is an engine that combines arbitrary elements according to nonlinear combining using a heuristic search method. In this example, the heuristic combining engine 24 uses an arbitrary (stochastic) combination of theoretical rules and/or heuristic rules to combine the existing data sets 81. A combining engine that uses a heuristic search method is disclosed for example in WO2002/087259. WO2002/087259 discloses a method that uses a genetic algorithm.

The Bayesian combining engine 23 combines the existing data sets 81 using Bayes' theorem (a Bayesian network). Methods of stochastically combining elements using a Bayesian network are known and are disclosed for example in WO00/075863 and WO2001/058145

Inference that may be used by the Bayesian combining engine 23, rules that may be used in the heuristic combining engine 24, and the like are stored in a library 29. The content of the library 29 is preferentially updated using the engines 23 and 24 according to the convergence state of the iterative algorithm engine 22.

Accordingly, in the processing unit 20, a plurality of virtual data sets 71 that are close to the IMS data 51 are generated based on the environment information 60 and the feature point information 19 of the IMS data 51 according to the environment in which the IMS data 51 was measured or conditions that are close to such environment. Since the virtual data sets 71 are generated by adding stochastic elements, compared to data sets that are theoretically or experimentally predicted, virtual data sets 71 have uncertainties or fluctuations. Accordingly, compared to data sets that are theoretically or experimentally predicted, virtual data sets 71 that are much closer to the IMS data 51 can be prepared.

In addition, since the virtual data sets 71 are generated in the processing unit 20 by adding stochastic and heuristic elements, even when the appearance pattern of objects cannot be predicted, when the environment information 60 has changed, when a change that cannot be express as a change in the environment information 60 has occurred, when there are limits on the environment information 60 obtained by the OLP 10, or when a change that cannot be predicted has occurred, there is still a high probability that virtual data sets 71 that are close to the IMS data 51 will be generated. Accordingly, it is possible to estimate objects of the IMS data 51 with much higher probability.

The iterative algorithm engine 22 generates virtual data sets 71 with the feature point information 19 of the IMS data 51 as a target. It is possible for the feature point information 19 to include the positions, forms (half-value width, height, dispersion, angle, and the like), peak intervals, and the like of the main peaks included in the spectra in the IMS data 51 and possible to control the amount of information of the target used by the iterative algorithm engine 22. Accordingly, it is possible to generate a number of virtual data sets 71 using the plurality of initial functions 25 and the initial conditions 26 that are stochastically generated and the amount of information in the feature point information 19 as the target. That is, in the iterative algorithm engine 22, the initial functions 25 and the initial conditions 26 generated by the stochastic combining engine 21 are used to combine the plurality of provisional candidate data sets 72 and/or existing data sets 81 according to an iterative algorithm with the feature point information 19 as a target so as to generate the virtual data sets 71. Accordingly, it is possible to stochastically find a plurality of virtual data sets 71 that are close to the IMS data 51.

In the iterative algorithm engine 22, it is also possible to increase the number of virtual data sets 71 by repeatedly using the iterative algorithm. That is, although a number of virtual data sets 71 are generated by an iterative algorithm (simulated annealing, mean field annealing, a genetic algorithm, or the like) with the feature point information 19 that has a small amount of information as a target, the iterative algorithm engine 22 may generate virtual data sets 71 using the same iterative algorithm again or a different iterative algorithm with feature point information 19 that has a comparatively large amount of information as the target and the generated virtual data sets 71 as initial values.

By doing so, in the OLP 10, a plurality of candidate data sets 75 that are groups of the plurality of virtual data sets 71 that have been stochastically generated by the processing unit 20 and one or a plurality of real data set 76 theoretically generated by the selection unit 40 are generated, thereby producing the candidate database 79. Accordingly, there is a high probability that virtual data sets 71 with a close Euclidean distance from the IMS data 51 will be included in the candidate database 79. For this reason, the output unit 30 is capable of determining the distances between the IMS data 51 and the respective candidate data sets 75 in the candidate database 79 having added more elements to the feature point information 19, such as the similarity of the spectra themselves, a background profile, or the like. This means that one or a plurality of candidate data sets 75 that are the objects of the IMS data 51 can be estimated with an even higher probability. The construction of the output unit 30 is the same as in the apparatus shown in FIG. 1.

The plurality of virtual data sets 71 included in the candidate database 79 are data sets that have been stabilized by the iterative algorithm engine 22 with the feature point information 19 as the target in a state with relatively little entropy, and do not include data sets where the distance (Euclidean distance) from the feature point information 19 is extremely large. However, although such data sets are close to the IMS data 51, they are still a provisional group that includes various data sets where the distance falls into different grades. For such information, the distances between such data sets also change if the objects being compared increase or the objects being compared change. Accordingly, even for a virtual data set 71 that is extremely close to the IMS data 51 in the range of the feature point information 19, if the distance from the IMS data 51 is re-evaluated by further considering minute peaks and other spectral characteristics that resemble noise that are not included in the feature point information 19 or that are deliberately excluded from the feature point information 19 due to a number of virtual data sets 71 being stochastically generated, there will be the possibility of the distance (ranking) from the candidate data sets 75 including such virtual data set 71 to the IMS data 51 changing. The selection unit 31 may include a function for this type of reevaluation.

The OLP 10 may also successively acquire the IMS data 51 and the environment information 60 at measurement intervals (sampling intervals) of the sensor 1. If a plurality of sensors 1 are connected to the interface 13, a plurality of IMS data 51 can be acquired by the OLP 10 at even shorter intervals. The selection unit 31 also includes a function that selects virtual data sets 71 that are close to the object on the fly from such successively acquired IMS data 51 and the virtual data sets 71 corresponding to such IMS data 51.

The one or plurality of candidate data sets 75 selected by the selection unit 31 is/are stored in the cache 39 and the most probable candidate data set(s) 75 is/are selected by the history searching unit 38. The information in the cache 39 may be fed back to the selection unit 31. That is, the OLP 10 receives a second measured data set (second IMS data) 51 that has been measured by the sensor (first sensor) 1 and second environment information 60 showing the measurement environment of the second IMS data 51 from the interface 13 and has virtual data sets 71 generated by the processing unit 20. The selection unit 31 further considers the history of the virtual data sets 71 determined to be close to the immediately previous or up to the immediately previous IMS data 51 and selects virtual data sets 71 that are close to the newly obtained IMS data 51 or generates a ranking of virtual data sets 71 for the newly obtained IMS data 51.

Typically, if a virtual data set 71 (candidate) has the common constituent or product to the constituent or product (candidate) of the virtual data set 71 that is close to the immediately previous or up to the immediately previous IMS data 51 and to the constituent or product (candidate) of virtual data set 71 that is close to the newly obtained IMS data 51 is given a high ranking as being close to the object. If the sensor 1 is placed in a closed environment (closed space), there is a high probability that IMS data 51 that is successively acquired by the OLP 10 will be a data set that has been previously measured for the same object. Accordingly, similarity to virtual data sets 71 that are close to IMS data 51 obtained immediately previously or obtained up to the immediately previous IMS data 51 is important when selecting objects.

On the other hand, if the sensor 1 is placed in an open environment, there is very low probability that IMS data 51 that is successively acquired by the OLP 10 will be data sets that have been previously measured for exactly the same object. In such OLP 10, the stochastic processing unit 20 will generate virtual data sets 71 including stochastic elements. Accordingly, there is a high probability of the virtual data sets 71 including, as candidates, data sets including constituents with a probability of being included in an open environment. For this reason, even when the sensor 1 is installed in an open environment, similarity to virtual data sets 71 that are close to IMS data 51 obtained immediately previously or obtained up to the immediately previous IMS data 51 is important when selecting objects.

The sensor control unit 32 may include a function for having the OLP 10 actively control the sensor 1 to improve the measurement precision for objects. As one example, if there are a plurality of candidate data sets 75 selected with similar rankings by the selection unit 31 for an object, it may be possible, by searching the spectrum information 82 of the existing data sets 81 combined in or included in the candidate data sets 75, to select one or a plurality of voltages Vf for separating such candidate data sets 75. The sensor control unit 32 feeds back the voltage Vf that is suited to changing the rankings of the plurality of candidate data sets 75 to the driver 11 as the measurement conditions 55, making it possible to change subsequent IMS data 51 to effective data for specifying the rankings of the candidate data sets 75 even more clearly.

In this way, in the OLP 10, virtual data sets 71 are generated by a prediction model and/or by adding stochastic elements, so as to increase the data sets 71 to be searched and widen the search space. By increasing the virtual data sets 71 to generate virtual data sets 71 that are close to the objects of the sensor 1 by incorporating stochastic and heuristic logic according to a prediction model and the environment information 60, it is possible to increase the virtual data sets 71 that are close to the IMS data 51 and at the same time prevent a situation where the search space is infinitely widened. As a result, there is an increase in virtual data sets 71 that are close to the IMS data 51 and there is a large reduction in the effective space to be searched, meaning that it is possible to select one or a plurality of constituents or products that are close to the object within a suitable time period and to display a ranking of such constituents or products.

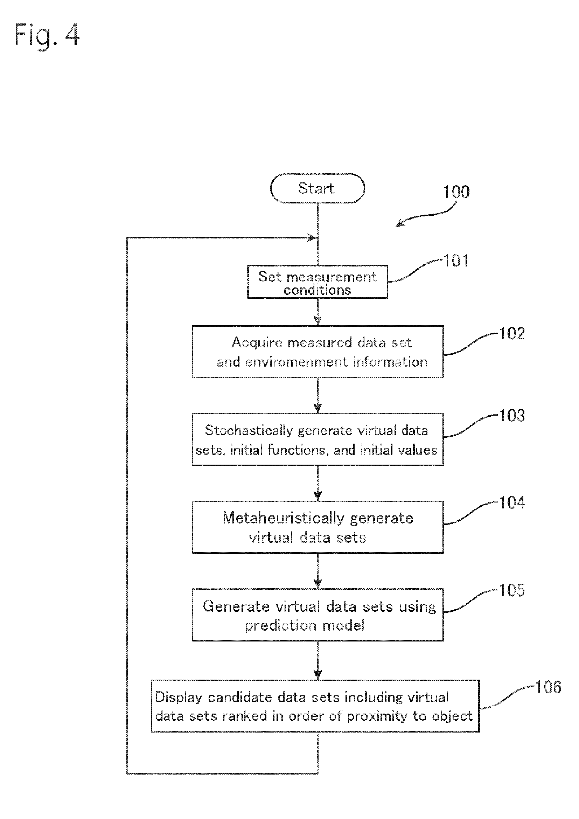

FIG. 4 shows one example of a method of estimating objects measured by the sensor 1 by way of a flowchart. In step 101, the measurement conditions 55 of the sensor 1 are set. In the case of a FAIMS sensor, by setting parameters that set the fundamental operation of the sensor, such as the field voltage Vf and the duty ratio and setting the variation range of the compensation voltage Vc, spectra showing the measured intensity of positively charged ions and negatively charged ions are obtained as measurement results.

One example of a measured data set (IMS data) 51 is spectra for a given voltage Vf. The IMS data 51 may be a combination of a plurality of spectra obtained at a plurality of voltages Vf or may be information produced by converting the characteristics of spectra to digital data. The setting of the measurement conditions 55 of the sensor 1 may be carried out by the OLP 10 or the terminal 2 may automatically set appropriate measurement conditions 55.

In step 102, the OLP 10 receives the IMS data 51 that is a data set measured by the sensor 1 and the environment information 60 from the terminal 2. The OLP 10 may be incorporated in the terminal 2 as described earlier. The environment information 60 should preferably include the many types of information included in the physical information 61 and the application information 62 described earlier. However, in addition to the generation of virtual data sets 71 using a prediction model, this method 100 and the OLP 10 include generating virtual data sets 71 using a stochastic means. This means that even if the amount of information included in the environment information 60 is limited or if hardly any environment information 60 can be obtained, there is still the possibility that it will be possible to generate virtual data sets 71 that match the environmental conditions in which the sensor 1 is carrying out measurement.

First, in step 103, the stochastic generation unit 220 generates the provisional candidate data sets 72, the initial functions 25, and the initial conditions 26 from the obtained environment information 60 and the existing data sets 81. Even in a situation where none of the sensor group 69 is installed in the terminal 2 and the physical information 61 cannot be obtained, the generation unit 220 is capable of estimating the physical information 61 to the greatest possible extent from the location information, time and date information, and the like included in the application information 62. Also, by asking the user of the terminal 2 questions such as "Is it hot or cold?", "What's the approximate temperature?", "How's the weather?", "Is it hot and humid?", it is possible to obtain low-precision physical information 61.

In step 104, the stochastic generation unit 220 also stochastically and heuristically generates the virtual data sets 75 using a metaheuristic iterative algorithm. Iterative algorithms include simulated annealing, mean field annealing, genetic algorithms, immune algorithms, and the like, and it is possible to use one or a combination of a plurality of such algorithms.

Steps 103 and 104 are examples where the stochastic generation unit 220 stochastically finds the virtual data sets 75. In step 103, a plurality of second converted data sets (provisional candidate data sets 72) are generated by combining a plurality of data sets that suit the first environment information 60 out of the plurality of existing data sets 81 with a relatively high probability, and in step 104, the plurality of second converted data sets 72 are combined via theoretic and/or heuristic parameters to stochastically discover a plurality of virtual data sets 71 with the feature point information 19 that is close to the first measured data set (IMS data) as the target.

Another example of a process for stochastic discovery may include:

- generating a plurality of first converted data sets in which the plurality of existing data sets 81 are theoretically and/or empirically changed based on the first environment information 60, and
- generating a plurality of virtual data sets 71 by stochastically combining a plurality of the first converted data sets.

The process of generating the plurality of virtual data sets 71 may include stochastically discovering a plurality of virtual data sets 71 that are close to the first measured data sets by combining a plurality of first converted data sets.

Another example of a process for stochastic discovery includes generating a plurality of virtual data sets 71 by combining a plurality of data sets that suit the first environment information 60 from a plurality of existing data sets 81 with a relatively high probability.

With the OLP 10, in step 105 the simulator 210 generates the plurality of virtual data sets 71 by converting the plurality of existing data sets 81 using a model that has the first environment information 60 as parameters. In this step 105, one or a plurality of suitable data sets out of the plurality of existing data sets 81 may be converted individually or as a combination by a prediction model to generate the virtual data sets 71.

The process of stochastically generating the virtual data sets 71 in step 103 and 104 may be executed in parallel with the process of generating the virtual data sets 71 using the prediction model in step 105, or one of such processes may be selected to generate the virtual data sets 71.

In step 106, the OLP 10 uses the output unit 30 so that one or a plurality of candidate data sets 75 that include the virtual data sets 71 can be displayed having been ranked in terms of proximity to the objects. In step 105, the plurality of candidate data sets 75 may be ranked based on information on the process that led to such candidate data sets 75, such as the convergence in the iterative algorithm engine 22, or the distances between each candidate data set 75 and raw or almost raw IMS data 51 (and not just the feature points) may be newly calculated and used to rank the candidate data sets 75 on display.

This estimation method 100 includes receiving the IMS data 51 measured by the sensor 1 (step 102), generating a plurality of virtual data sets by stochastically combining a plurality of existing data sets 81 (steps 103 and 104), and selecting at least one candidate that is close to the IMS data 51 out of provisional groups that include the plurality of candidate data sets 75 (step 105).

Although not limited to a sensor such as a DMS that measures physical amounts based on ion mobility, the appearance pattern of characteristics of the object to be measured in the data set obtained by a sensor is affected by various elements. Accordingly, there is low probability that a data set that has been affected by such elements will be included in the existing data sets. In addition, there is low probability that a data set showing the object to be measured included in existing data sets will be closest to measured data that has been affected by various factors. It may be possible to estimate or predict an appearance pattern according to the various elements of the data set showing the object to be measured included in existing data sets. However, it is rare for all of the elements that affect the appearance pattern to be obtained in advance. It is also rare for all the elements that affect the appearance pattern and the effects caused by such elements to be clearly understood.

In this estimation method 100, search objects (a search space) are virtually widened by stochastically combining a plurality of existing data sets, virtual data sets that are close to the measured data set are stochastically increased, and the measured object or a virtual data set that is close to the measured object is disposed closed to the measured data set. By then searching such a search space, there is the possibility of obtaining the measured object or a candidate that is close thereto and possible to improve the estimation probability for measured objects even if all of the elements that affect the appearance pattern cannot been obtained in advance and all of the elements that affect the appearance pattern and the effects due to such elements are not all clearly understood.

In addition, with this method, a step of receiving the environment information 60 showing the measurement environment of the measured data set is provided and the process of generating the plurality of virtual data sets includes generating the plurality of virtual data sets 71 by combining the existing data sets 81 with probabilities controlled according to the environment information 60. Since there is the probability that the range of generating the virtual data sets 71 can be limited by the environment information 60, it is possible to narrow the search space and to reduce the time required to estimate the objects.

Although the processing apparatus 10 and the estimation method 100 are examples where a FAIMS is used as a sensor, the sensor is not limited to a FAIMS and it is also possible to apply the processing apparatus 10 and the estimation method 100 to a DMS or another type of IMS, such as a TOF (time of flight) IMS. In addition, the processing apparatus 10 and the estimation method 100 can be widely applied to systems that require measurement by a sensor that often measures the output of a system in which a number of elements or factors are in a complex relationship and estimation of the objects being measured.

The processing apparatus 10 may be present on a network, may be installed in an apparatus such as the terminal 2 together with the sensor 1, and the processing apparatus 10 may be equipped with the database 80 including the existing data sets 81. The estimation method 100 and the processing apparatus 10 can be implemented in hardware such as an LSI as hardware logic. The estimation method 100 and the processing apparatus 10 can also be recorded on a suitable recording medium or provided via a network as software (a program or a program product) so as to enable execution by a computer with suitable hardware resources.

The processing apparatus 10 and the method 100 (hereinafter simply "OLP") has a search performance that is favorable with respect to variations in environmental parameters (such as temperature, humidity, pressure, and flow rate). Accordingly the processing apparatus 10 and the method 100 can be used in a variety of applications including security and process control and monitoring. The OLP provides a database management system including registration and management of new chemical substances. In addition, the OLP includes a function as an analysis/search engine module and provides a GUI-based analysis/search engine for simplifying detection of foreign matter during continuous monitoring. The OLP also includes a function as a data collection module equipped with a function that customizes the data sampling points so as be optimal for the substances to be detected. The OLP also provides an API that is compatible with various algorithms for preprocessing and analysis processing. Accordingly, it will be possible to expand the functioning by way of an analysis algorithm that is developed in the future or is provided by the user. In addition, the OLP is also capable of providing an API suited to cloud computing and makes it possible to construct a server-client system that uses the Internet.

The OLP can be applied in various fields and is favorable for real time analysis and detection, threat detection, analysis of fragrances and smells, specifying trace chemicals, and the like. Such fields include food processing and process monitoring, security, the fragrance/perfume business, medicine and the medical field, the research field for olfactory processing, the field of water treatment, and development of entertainment content. As one example, in the fields of food and beverage quality control and quality control of pharmaceutical products, it is important to detect and identify substances that pose dangers to foodstuffs and chemical substances that have safety problems, making it desirable to solve such problems using an OLP.

In the field of security, it is important to carry out detection reliably with processing of a certain speed or faster, and by applying an OLP, there is possibility of reducing errors and improving the detection reliability and precision. In addition, the detection of explosive substances and hazardous substances, such as in anti-terrorism, requires a function for efficient registration and management in a short time by updating or creating a database for the latest information at all times. An OLP has high flexibility and the potential to satisfy such demands, including feedback from an actual application site.

In the healthcare market and the field of monitoring indoor air conditioners, in breath monitoring for example, a causal relationship has been established between certain illnesses and incurable diseases and breath that results from biological reactions, with it being possible to easily provide a monitoring function by applying an OLP. In the field of other consumer applications, OLP enable product development and business opportunities based on semiconductor device technology, and can provide a variety of users with analysis technology that is easy to use.

**Claims**

1.  An apparatus comprising:

    an interface that receives a first measured data set that is measured by a first sensor and first environment information showing a measurement environment of the first measured data set;
    a generation unit that generates a plurality of virtual data sets by converting a plurality of existing data sets from a database using conditions controlled according to the first environment information; and
    an output unit that selects and outputs at least one candidate that is close to the first measured data set from a provisional group including the plurality of virtual data sets.

2.  The apparatus according to claim 1:

    wherein the generation unit generates the plurality of virtual data sets by combining the plurality of existing data sets acquired from the database with a probability controlled according to the first environment information.

3.  The apparatus according to claim 1 or claim 2,
    wherein the generation unit includes:

    a unit that generates a plurality of first converted data sets produced by theoretically or experimentally changing the plurality of existing data sets based on the first environment information; and
    a unit that stochastically finds a plurality of virtual data sets that are close to the first measured data set by combining the plurality of first converted data sets.

4.  The apparatus according to any of claim 1 to claim 3,
    wherein the generation unit includes a unit that generates the plurality of virtual data sets by combining a plurality of data sets, out of the plurality of existing data sets, that are suited to the first environment information with relatively high probabilities.

5.  The apparatus according to any of claim 1 to claim 4,
    wherein the generation unit includes:

    a unit generating a plurality of second converted data sets by combining a plurality of data sets, out of the plurality of existing data sets, that are suited to the first environment information with relatively high probabilities; and
    a unit that stochastically finds a plurality of virtual data sets that are close to the first measured data set by combining the plurality of second converted data sets via theoretical and/or experimental parameters.

6.  The apparatus according to any of claim 1 to claim 5,
    wherein the generation unit includes a simulator that generates a plurality of virtual data sets by converting the plurality of existing data sets using a model that has the first environment information as parameters.

7.  The apparatus according to claim 6,
    wherein the simulator includes a unit that selects a plurality of data sets suited to the first environment information out of the plurality of existing data sets for conversion by the model.

8. The apparatus according to claim 6 or claim 7,
   further comprising a unit that extracts feature points of the first measured data set,
   wherein the simulator includes a unit that selects, out of the plurality of existing data sets, a plurality of data sets that share the feature points for conversion by the model.

9. The apparatus according to any of claim 1 to claim 8,
   wherein the output unit includes a unit that outputs, from the interface, a candidate that is common to both:

   at least one candidate that is close to a second measured data set measured by a first sensor and a second measured data set selected according to second environment information showing a measurement environment of the second measured data set; and
   at least one candidate that is close to the first measured data set.

10. The apparatus according to any of claim 1 to claim 9,
    wherein the first environment information includes at least one of data showing the measurement environment of a first sensor measured by a second sensor, position information measured by a third sensor, and application information inputted via a camera or an external input interface.

11. The apparatus according to any of claim 1 to claim 10,
    further comprising a sensor control unit outputting measurement conditions to the first sensor.

12. The apparatus according to any of claim 1 to claim 11,
    further comprising the first sensor.

13. The apparatus according to any of claim 1 to claim 12,
    further comprising the database.

14. The apparatus according to any of claim 1 to claim 13,
    wherein the output unit outputs product names of the candidates.

15. A program causing a computer to function as the apparatus according to any of claim 1 to claim 14.

16. A method of having a processing apparatus process data measured by a first sensor, comprising:

    the processing apparatus receiving a first measured data set that is measured by the first sensor;
    the processing apparatus receiving first environment information showing a measurement environment of the first measured data set;
    the processing apparatus generating a plurality of virtual data sets by converting a plurality of existing data sets using conditions controlled according to the first environment information; and
    the processing apparatus selecting at least one candidate that is close to the first measured data set from a provisional group including the plurality of virtual data sets.

17. The method according to claim 16,
    wherein generating the plurality of virtual data sets includes generating the plurality of virtual data sets by combining the plurality of existing data sets with a probability controlled according to the first environment information.

18. The method according to claim 16 or claim 17
    wherein generating the plurality of virtual data sets includes:

    generating a plurality of first converted data sets produced by theoretically or experimentally changing the plurality of existing data sets based on the first environment information; and
    generating a plurality of virtual data sets by stochastically combining the plurality of first converted data sets.

19. The method according to claim 18,
    wherein generating the plurality of virtual data sets includes stochastically finding a plurality of virtual data sets that are close to the first measured data set by combining the plurality of first converted data sets.

**20.** The method according to any of claim 16 to claim 19,
wherein generating the plurality of virtual data sets includes generating the plurality of virtual data sets by combining a plurality of data sets, out of the plurality of existing data sets, that are suited to the first environment information with relatively high probabilities.

**21.** The method according to any of claim 16 to claim 20,
wherein generating the plurality of virtual data sets includes:

> generating a plurality of second converted data sets by combining a plurality of data sets, out of the plurality of existing data sets, that are suited to the first environment information with relatively high probabilities; and stochastically finding a plurality of virtual data sets that are close to the first measured data set by combining the plurality of second converted data sets via theoretical and/or experimental parameters.

**22.** The method according to any of claim 16 to claim 21,
wherein generating the plurality of virtual data sets includes generating a plurality of virtual data sets by converting the plurality of existing data sets using a model that has the first environment information as parameters.

**23.** The method according to claim 22,
wherein generating a plurality of virtual data sets by converting using the model includes generating a plurality of virtual data sets by converting a plurality of data sets suited to the first environment information out of the plurality of existing data using the model.

**24.** The method according to claim 22 or claim 23,
wherein generating a plurality of virtual data sets by converting using the model includes:

> extracting feature points of the first measured data set; and
> generating a plurality of virtual data sets by converting a plurality of data sets, out of the plurality of existing data sets, that share the feature points using the model.

**25.** The method according to any of claim 16 to claim 24, further comprising:

> receiving a second measured data set measured by the first sensor;
> receiving second environment information showing a measurement environment of the second measured data set;
> generating a plurality of virtual data sets by converting a plurality of existing data sets using conditions controlled according to the second environment information;
> selecting at least one candidate that is close to the second measured data set out of a provisional group including the plurality of virtual data sets; and
> selecting a candidate that is common to at least one candidate that is close to the first measured data set and at least one candidate that is close to the second measured data set.

Fig. 1

Fig. 2

(a)

(b)

Fig. 3

# Fig. 4

Start — 100

Set measurement conditions — 101

Acquire measured data set and enviromenment information — 102

Stochastically generate virtual data sets, initial functions, and initial values — 103

Metaheuristically generate virtual data sets — 104

Generate virtual data sets using prediction model — 105

Display candidate data sets including virtual data sets ranked in order of proximity to object — 106

## INTERNATIONAL SEARCH REPORT

| International application No. |
|---|
| PCT/JP2011/004884 |

**A. CLASSIFICATION OF SUBJECT MATTER**
*G01N27/62*(2006.01)i

According to International Patent Classification (IPC) or to both national classification and IPC

**B. FIELDS SEARCHED**

Minimum documentation searched (classification system followed by classification symbols)
G01N27/60-27/70

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2010-506150 A (Smiths Detection Inc.), 25 February 2010 (25.02.2010), claims 1, 14 to 21; paragraphs [0017] to [0020], [0043], [0051] & EP 2069779 A      & WO 2008/039996 A2 | 1-25 |
| X | JP 2006-53004 A (Hitachi High-Technologies Corp.), 23 February 2006 (23.02.2006), claims; paragraphs [0025] to [0045] (Family: none) | 1-25 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 22 November, 2011 (22.11.11) | 06 December, 2011 (06.12.11) |

| Name and mailing address of the ISA/ Japanese Patent Office | Authorized officer |
|---|---|
| Facsimile No. | Telephone No. |

Form PCT/ISA/210 (second sheet) (July 2009)

## INTERNATIONAL SEARCH REPORT

International application No.

PCT/JP2011/004884

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2007-513340 A  (Sionex Corp.),<br>24 May 2007 (24.05.2007),<br>paragraphs [0069] to [0071], [0124] to [0131],<br>[0212]<br>& US 2005/0139762 A1     & EP 1690074 A<br>& WO 2005/052546 A2 | 1-25 |
| A | JP 2005-513414 A  (Sionex Corp.),<br>12 May 2005 (12.05.2005),<br>claims 1, 2; paragraph [0175]<br>& US 2003/0052263 A1     & EP 1405065 A<br>& WO 2003/005016 A1 | 1-25 |
| P,X | WO 2011/077731 A1  (Atonarp Inc.),<br>30 June 2011 (30.06.2011),<br>paragraphs [0073], [0074]<br>(Family: none) | 1-25 |

Form PCT/ISA/210 (continuation of second sheet) (July 2009)

| INTERNATIONAL SEARCH REPORT | International application No. |
| --- | --- |
| | PCT/JP2011/004884 |

Claims 1 – 25 contain a means for selecting a candidate with respect to every measurement data which is not specified data to be targeted wherein the candidate for "a data set" which is "measured by a sensor" is selected.  However, only an ion mobility spectrometer or an electrical mobility spectrometer that is specified in the description is disclosed within the meaning of PCT Article 5, and thus claims 1 through 25 are not fully supported within the meaning of PCT Article 6.

That is, since a correction method varies greatly depending on the measurement means, a point in that data provided by other measurement methods "is converted with a condition controlled by environment information" is not considered to be supported by the description.

This international search report therefore covers those supported and disclosed by the description, namely data specifically mentioned in the description wherein the data is measured by a specific sensor (measurement device).

Form PCT/ISA/210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008508693 A
- WO 2006013396 A
- JP 2007513340 A
- WO 2005052546 A
- WO 2002087259 A
- WO 00075863 A
- WO 2001058145 A